# EUROPEAN PATENT SPECIFICATION

(11) **EP 0 317 254 B1**
(45) Date of publication and mention of the grant of the patent: **29.09.1993**
(21) Application number: 88310755.9
(22) Date of filing: 15.11.1988
(51) Int. Cl.: C12N 15/81, C12N 15/63, C12N 1/16

(54) **Yeast expression system**
Hefe-Expressionssystem
Système d'expression pour levure

(30) Priority: 18.11.1987 GB 8726953
(43) Date of publication of application: 24.05.1989
(73) Proprietor: Delta Biotechnology Limited, Nottingham NG7 1FD (GB)
(72) Inventor: Kenny, Enda, Leicestershire (GB); Hinchliffe, Edward, Nottinghamshire (GB)
(74) Representative: Bassett, Richard Simon

(56) References cited:
- EP-A- 0 132 309
- PROCEEDINGS OF THE NATIONAL ACADEMY OF SCIENCE USA, vol. 79, December 1982, pages 7410-7414; L. GUARENTE et al.: "A GAL10-CYC1 hybrid yeast promoter identifies the GAL4 regulatory region as an upstream site"
- BIOTECHNOLOGY AND GENETIC ENGINEERING REVIEWS, vol 3, September 1985, pages 377-416, Intercept Ltd., Newcastle-Upon-Tyne, GB; S.M. KINGSMAN et al.: "Heterologous gene expression in saccharomyces cerevisiae"
- St John & Davis (1981) J. Mol. Biol. 152, 285-315
- Struhl (1984) Proc. Natl. Acad. Sci. USA 81, 7865-7869
- Hinnebusch et al (1985) Proc. Natl. Acad. Sci. USA 82, 498-502
- Guarente & Hoar (1984) Proc. Natl. Acad. Sci. USA, 81, 7860-7864
- Ogden et al (1986) Mol. Cell. Biol. 6, 4335-4343
- Sarokin & Carlson (1986) Mol. Cell. Biol. 6, 2324-2333
- Lowry et al (1983) Proc. Natl. Acad. Sci. USA 80, 151-155

## Description

This invention relates to the field of recombinant DNA technology. In particular it relates to a novel yeast expression system.

In yeast, gene expression is controlled, at least partly, by promoters which lie upstream of the structural genes or coding regions themselves. Several yeast promoters have been isolated and used to express foreign gene products in yeast. The promoters have the characteristics of the gene product they would normally express. Phosphoglycerate kinase (PGK), for example, is an essential enzyme and its promoter is therefore constitutive although somewhat elevated in the presence of fermentable carbon sources such as glucose (Mellor et al., 1983). The PGK promoter has been used to express a variety of heterologous polypeptides in yeast. (Kingsman et al., 1986).

It may be beneficial in a process to regulate the expression of a heterologous polypeptide, particularly if that product is harmful to the cell or rapidly degraded once synthesised. Under these circumstances an inducible gene expression system is desired. One of the best studied regulated systems in yeast is the one controlling the expression of the galactose catabolic enzymes.

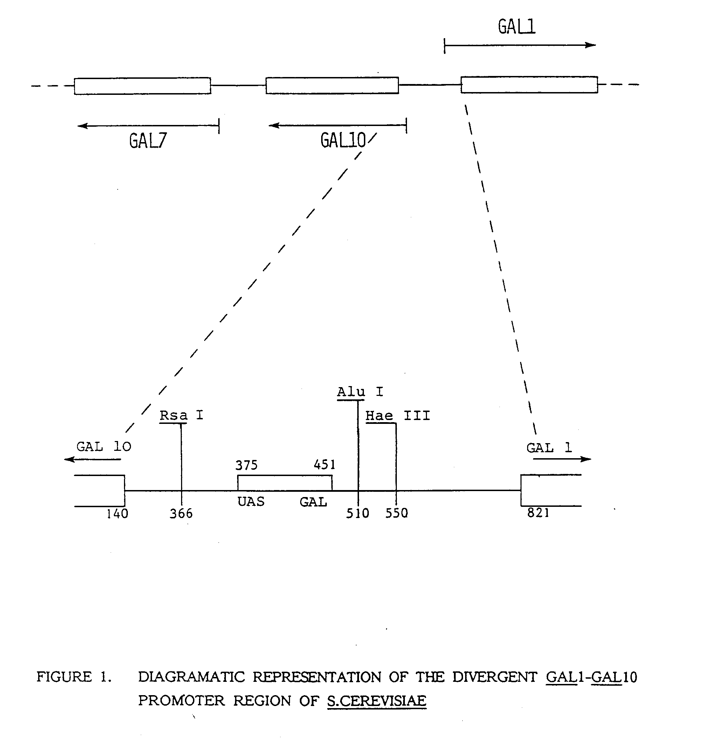

In the absence of galactose, the catabolic enzymes encoded by genes GAL 1, 7 and 10 are not synthesised. Upon addition of galactose and in the absence of glucose, these genes are rapidly activated and the level of transcription is 1,000 fold more than the uninduced levels (Hopper et al., 1978; St. John and Davis, 1979). The activation is effected by a regulator protein, encoded by the GAL4 gene. The presence of galactose allows GAL4 to bind upstream of the structural genes and thus promote expression. The site of this binding is termed the upstream activation sequence or UAS (Guarante et al., 1982). The chromosomal arrangement of the catabolic genes is shown in Figure 1 (St. John and Davis, 1981; St. John et al., 1981). A single UAS controls the divergent transcription of GAL1 and GAL10

It has been shown that substitution of the native UAS of a promoter by the GAL10 UAS confers galactose inducibility upon that promoter (Guarante et al., 1982). It has also been found (see our earlier co-pending patent application EP-A-258 067) that the GAL10 UAS used in the construction of a PGK: GAL10 hybrid promoter, inducible with galactose, functions in either orientation, that is to say that a linear DNA fragment containing the GAL10 UAS may be substituted for the native PGK UAS and confer galactose regulation regardless of the orientation of the DNA fragment.

It has furthermore been suggested (EP-A-132 309), although not demonstrated, that the (GAL10 promoter-UAS-GAL1 promoter) entity is a portable, divergent regulatory element suitable for use with, and sandwiched between, two heterologous coding regions. What has not been shown, and could not have been predicted, is that the GAL UAS itself will still function as a divergent element even if the respective promoters on each side of it are not the promoters which are native to it.

The GAL 1/10 UAS has been shown not to co-ordinately regulate the transcription of two heterologous divergent transcripts (Struhl, 1984).

We have found that the GAL UAS can be used to control the divergent transcription of two heterologous promoters in a hybrid system; when the GAL UAS is inserted appropriately between two promoters that lack a UAS sequence, transcription has been found to be regulated in both promoters by the presence of galactose, and divergent transcription is achieved.

Accrodingly, one aspect of the present invention provides a DNA sequence comprising the GAL UAS located between first and second transcription promoters, the said two promoters each being heterologous to the said UAS, in mutually opposite orientation and operably linked to the said UAS such that the UAS is capable of activating transcription from each said promoter, each said promoter being derived from a gene which has a native UAS, the said native UAS being absent in the said DNA sequence.

The invention also provides such an element in conjunction with coding regions such that each promoter is located between the GAL UAS and a respective said coding region for a desired polypeptide. The promoters and coding regions are of course in proper reading frame and in such an orientation that the two coding regions may be transcribed in respective opposed directions under the control of their respective promoters to provide mRNA encoding the desired polypeptides.

The promoters which are used will generally have their respective UAS's (if present) deleted. In some cases, a UAS-like region which is rich in A's and T's but which does not bind a regulatory protein can be left, but in other cases it should be deleted. The skilled man will be able to make such modifications without exercising inventive effort.

It is to be understood that, in accordance with the known requirements for efficient transcription and translation, appropriate stop signals, capping sequences and the like will usually be included when sequences in accordance with the invention are to be used in polypeptide-producing constructs. The two coding regions should not be GAL1 and GAL10, respectively.

The GAL UAS has been defined in the art, in particular in Giniger et al, 1985, Cell 40, 767-774.

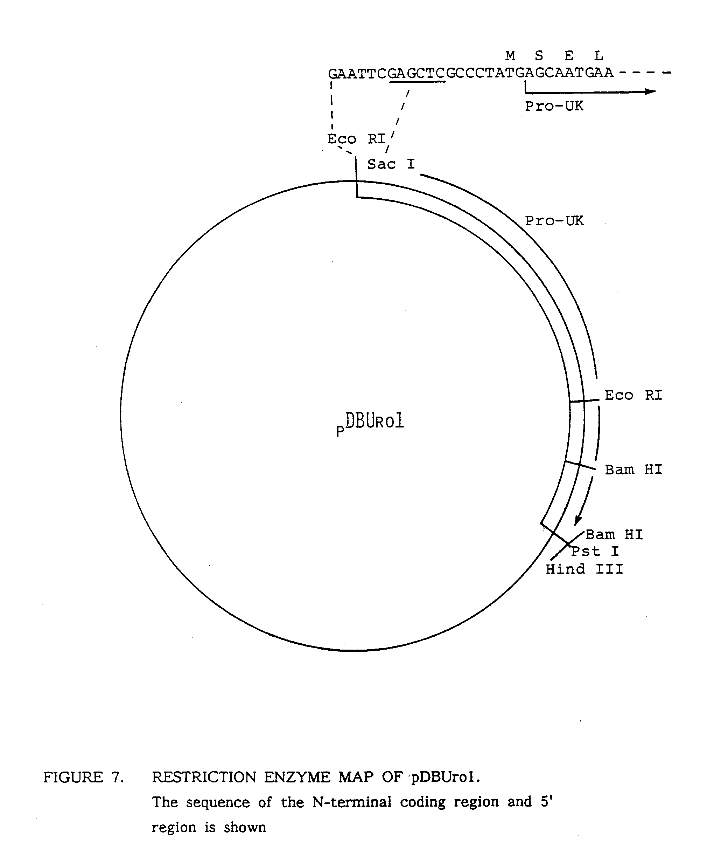

The UAS functions as a binding site for an activator protein, the product of the GAL4 gene. The GAL UAS has been defined as comprising four homologous 17 base pair sequences exhibiting dyad symmetry. It is these sequences which direct the binding of the GAL4 protein within this region. One such element upstream of a promoter is sufficient to activate transcription although maximal levels are achieved with multiple copies of the element. Thus, as used herein, the term "GAL UAS" encompasses any sequence which will bind the product of the GAL4 gene and/or which is one or more of the said 17 base pair sequences, the consensus or near consensus sequences therefor or synthetic analogues thereof such as are disclosed in Giniger et al, loc. cit., especially Figure 7 and the legend therefor. If more than one 17 base pair sequence is present, they may be the same or different. It is preferred for the UAS not to consist of only the fourth 17 base pair sequence identified by Giniger et al, or repeats thereof, as (on its own) it appears not to bind the GAL4 protein very strongly.

The said two coding regions may be the same or different. In a preferred embodiment, one coding region encodes human serum albumin (HSA) and the other encodes urokinase. Pro- or pre-pro-sequences may be included as appropriate. When the two polypeptides are different, it may be desirable for one polypeptide to be secreted from a host micro-organism harbouring the DNA sequence of the invention, and for the other polypeptide to be produced and stored intra-cellularly, so that purification of the two polypeptides is facilitated. Thus, the DNA sequence may encode (i) pro-HSA preceded by a suitable signal sequence, for example the native HSA signal sequence or the yeast alpha-factor signal sequence, and also (ii) pro-urokinase, without any signal sequence.

One or more further coding regions may be included, under the control of either of the said promoters, to provide fusion proteins.

Further aspects of the invention include: (a) a vector comprising the said DNA sequence and suitable for transforming a microorganism; (b) a plasmid comprising the said DNA sequence and capable of self-replication in a suitable host bacterium, alga, fungus (including yeast) or other eucaryotic (for example mammalian) cell; (c) a host microorganism (i.e. a bacterium, alga, fungus or other eucaryotic cell) transformed with the said DNA sequence, either on a self-replicating plasmid or integrated into the chromosome; (d) a method of preparing a DNA sequence as defined above by appropriate manipulation of and/or synthesis of DNA fragments comprising one or more parts of or the whole of the GAL UAS, the said promoters and the said coding regions; (e) a process for producing one or more polypeptides by fermentation of a microorganism according to (c) above in a suitable medium including sufficient galactose to activate the GAL UAS; and (f) a polypeptide when produced according to process (e) above.

If yeast is the host microorganism listed as (c) above, then the elements of the galactose regulatory system, such as the GAL4 gene, will normally be present and thus the GAL UAS may be acted upon in the usual way. In other hosts, it may be necessary to transform the host with such elements, so that galactose-induced transcription of the coding regions referred to above may occur. Transformation of mammalian cells with the yeast GAL4 gene has been demonstrated by Kakidani & Ptashne, 1988, Cell, 52, 161-167 and by Webster et al, 1988, Cell, 52, 169-178. Such methods are taught generally in the chapter by Gorman in "DNA Cloning" Volume III, (Ed) D. Glover, 1986. The GAL4 gene may be cloned into the host cell on the same vector as the promoter-UAS-promoter sequence of the invention, or on another vector. Once in the host, the GAL4 gene may integrate with the chromosome or be on a self-replicating plasmid. The GAL4 gene will be under the control of a suitable constitutive or inducible promoter compatible with the host cell, for example the mouse mammary tumour promoter (MMTV) in a mammalian cell line. (Scheidereit et al, 1983, Nature 304, 749-752).

In a particularly preferred embodiment of process (e) above, the microorganism (for example laboratory yeast or brewing yeast) is capable of being thus secreted and is purified from the fermentation medium, and the second polypeptide is produced only intra-cellularly and is purified from broken cells of the microorganism, the latter having been separated from the fermentation medium.

Regulation of transcription can be achieved using yeast hosts which provide repressors controlled by chemical or physical means. Various co-repressors for the GAL genes include glucose, glycerol, and the product of GAL80 gene. Positive regulation or activation can be achieved with inducers, such as galactose, or activators, such as the product of the GAL4 gene. Rather than using chemical regulation, one can employ temperature-sensitive mutations, which allow for regulation through repression and/or activation, by having temperature shifts, either up or down, generally in the range of from about 20°C to 50°C.

Extending downstream from the promoters may be a wide variety of flanking sequences, providing numerous functions. Once can provide for cohesive ends or blunt ends to the fragment, so as to be capable of ligation to other DNA sequences, depending upon particular situations. One can cleave immediately downstream from the promoter, so that one would join the divergent promoter function DNA sequence to other sequences which had the necessary transcriptional and translational signals.

Optionally, one can have a terminus at a site downstream from the promoter where all of the transcriptional and translational regulatory signals downstream from the promoter and associated therewith have been removed. Alternatively, one can provide for retaining all of those transcriptional and translational regulatory sequences downstream from the promoter, but remove the initiation codon and, optionally, a few bases upstream from the initiation codon, generally not more than about 20 bases, conveniently not more than 10 bases.

Finally, one may cleave in the coding region naturally associated with the promoters at a site which will permit joining of a DNA sequence in frame with the respective initiation codons. In the last situation, one will obtain a fused protein.

An advantage of using the GAL UAS according to the invention, instead of using separate UAS's, is that the relative levels of the two mRNA's and/or polypeptides may be fixed, for example at 1:1. This may be useful when the two polypeptides are subunits of the same protein or enzymes in the same metabolic pathway. Furthermore, the presence of only one UAS for the two coding region/promoter systems tends to increase the stability of the plasmid and to reduce the incidence of recombination. The two promoters may be more productive or otherwise more suited to the expression of the coding region than the GAL 1 and GAL10 promoters of EP-A-132 309.

The following Examples illustrate preferred embodiments of the invention, with reference to the accompanying drawings, in which:
Figure 1 is a diagramatic representation of the divergent GAL1-GAL10 promoter region of S. cerevisiae;
Figure 2 shows the construction of UAS GAL plasmids pDB1 and pDB2;
Figure 3 is a restriction enzyme map of plasmid pKV50;
Figure 4 is a restriction enzyme map of pEX-2 (from Ernst and Chan, 1985; Gatignol et al. 1987);
Figure 5 is a restriction enzyme map of pEK30;
Figure 6 is a restriction enzyme map of pEK113 (Hinchliffe and Kenny, 1986), where the black block represents pBR322 DNA and the open block represents met-HSA DNA;
Figure 7 is a restriction enzyme map of pDBUro1, where the sequence of the N-terminal coding region and 5′ region is shown;
Figure 8 is a restriction enzyme map of pEK31;
Figure 9 is a restriction enzyme map of pSJ22;
Figure 10 is a schematic representation of a Western blot demonstrating the intracellular expression of pro-urokinase and HSA in pSJ22. Lanes 1 to 4 and 5 to 8 represent immunoblots using pro-urokinase and HSA antisera respectively. The order of loading is as follows:-
   Lanes 1 and 5; Induced, soluble cell fraction
   Lanes 2 and 7; Induced, insoluble cell fraction
   Lanes 3 and 6; Uninduced, insoluble cell fractions
   Lanes 4 and 8; Urokinase and HSA standard, respectively;
Figure 11 is a restriction enzyme map of pET13:1 (Henderson et al 1985);
Figure 12 is a restriction enzyme map of pSJ22a;
Figure 13 shows the nucleotide sequence of a 255 base pair BamH1-HindIII fragment encoding a partial MF alpha-1 pre-pro sequence. This was synthesised by ligation of the synthetic oligonucleotide duplexed 1-1a, 2-2a and 3-3a. The solid lines delineate the individual oligonucleotides. This fragment was then inserted between the BamHI and HindIII sites of M13mp19 to yield mp19 alpha;
Figure 14 illustrates the construction of plasmid mp19.7 alpha 1;
Figure 15 illustrates the construction of plasmid mp19 alpha 2;
Figure 16 shows the structure of pEK44; and
Figure 17 is a restriction enzyme map of pEK72, in which the HSA transcript is an MF-alpha1 fusion such that the resultant polypeptide will be secreted. u-PA is N-methionyl and will be retained intra-cellularly.

### EXAMPLE I : LABORATORY YEAST - HSA/UROKINASE

The strategy for the construction of the DNA sequence of the invention was as follows. First, the PGK UAS was deleted and substituted with the GAL UAS. This was accomplished in such a way that a unique restriction site was placed immediately 5′ to the UAS and distal to the PGK promoter. Finally, the CYC-1 promoter was inserted at the unique site in an orientation such that transcription would be divergent with respect to that directed by the PGK promoter. The final construction also contained unique restriction sites suitable for the insertion of heterologous genes for expression.

Standard methods were used in the manipulation of DNA (Maniatis et al. (1982)). Restriction enzymes and other enzymes were used according to the manufacturers' instructions. Oligonucleotides were synthesised, using phosphoramidite chemistry, on an Applied Biosystems 380B machine using reagents supplied by the manufacturer (Applied Biosystems Inc., Foster City, California).

Other general methods such as transformation of E.coli and yeast strains, preparation of cell extracts, SDS electrophoresis and immunoblotting were as described by Hinchliffe and Kenny (1986).

### Deletion of the PGK UAS

The deletion of the PGK UAS was accomplished by enzymatic digestion of the 5′ non-coding region of the PGK gene. The deletion was carried out bidirectionally as follows. First, 5′ to 3′ deletions were obtained in a derivative of plasmid pMA27 (Mellor et al., 1983) in which the ClaI site at position -800 in the PGK 5′ non-coding region had first been converted to a unique XhoI restriction site using a synthetic oligonucleotide linker. This pMA27 derivative was then cleaved with XhoI and digested with BAL31 exonuclease. Plasmids were recircularised by ligation in the presence of BamHI synthetic oligonucleotide linkers and transformed into E.coli. The deletion end points were characterised by DNA sequencing. Deletions in the 3′ to 5′ direction were made by similar modification of pMA22a (Dobson et al., 1982), only in this latter case the plasmid was digested with BamHI prior to BAL31 exonuclease treatment. As before, the deletion end points were determined by DNA sequence analysis.

Plasmid pDB4 was constructed by ligating the appropriate BamHI-PstI fragments from the 5′ and 3′ deletion derivatives. Plasmid pDB4 has a deletion between co-ordinates -423 and -470 which includes the PGK UAS itself. Plasmid pDB4 is therefore identical to the parent plasmid pMA27 with the exception of the deletion in the 5' non-coding region of the PGK gene. When transformed into yeast this vector only confers slightly above host chromosomal levels of PGK protein despite being in the multicopy state.

### Construction of a Galactose Inducible PGK Gene

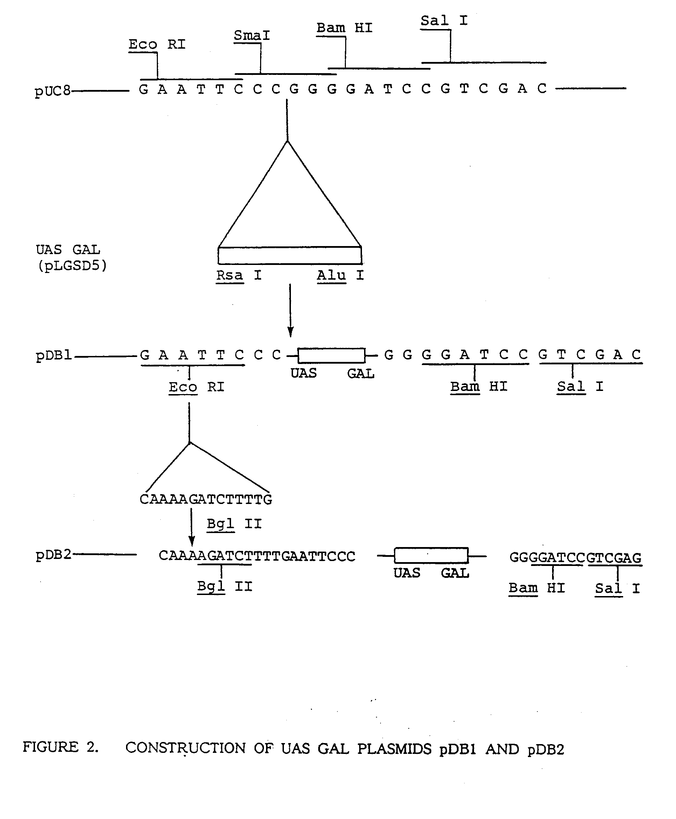

The organisation of the GAL1-GAL 10 divergent promoter is shown in Figure 1. The functional region of the UAS has been localised (West et al., 1984) and its position is indicated along with flanking restriction sites. The GAL10 UAS may be found on plasmid pLGSD5 (Guarante et al., 1982). The 144bp RSaI-AluI DNA fragment from pLGSD5 was isolated and blunt end ligated with the SmaI site of pUC8 to form pDB1 (Fig. 2). Subsequently the unique EcoRI site of pDBI was converted to a BglII site by the insertion of a BglII oligonucleotide linker (Figure 2). Thus the GAL10 UAS could be isolated on a unique BglII-BamHI DNA fragment. This fragment was subsequently cloned into the unique BamHI site in pDB4. The resultant plasmid, pKV44, was shown to encode a galactose regulatable PGK gene. In the absence of galactose, similar levels of PGK were found in yeasts harbouring pKV44 as pDB4. However, upon addition of galactose and in the absence of glucose, expression was greatly increased in strains harbouring pKV44 whilst in stains harbouring pDB4 expression levels of PGK were unchanged.

### Construction of GAL10: PGK Expression Vector

Plasmid pDB4 was digested at the unique BglII site located in the 3' region of the PGK structural gene and the linear molecule was digested with Bal31 exonuclease. DNA fragments were filled in and ligated in the presence of BglII oligonucleotide linkers and the plasmids thus formed were screened by DNA sequence analysis to determine the deletion end point. Amongst others, a deletant with an end point at position -8 with respect to the PGK coding sequence was obtained.

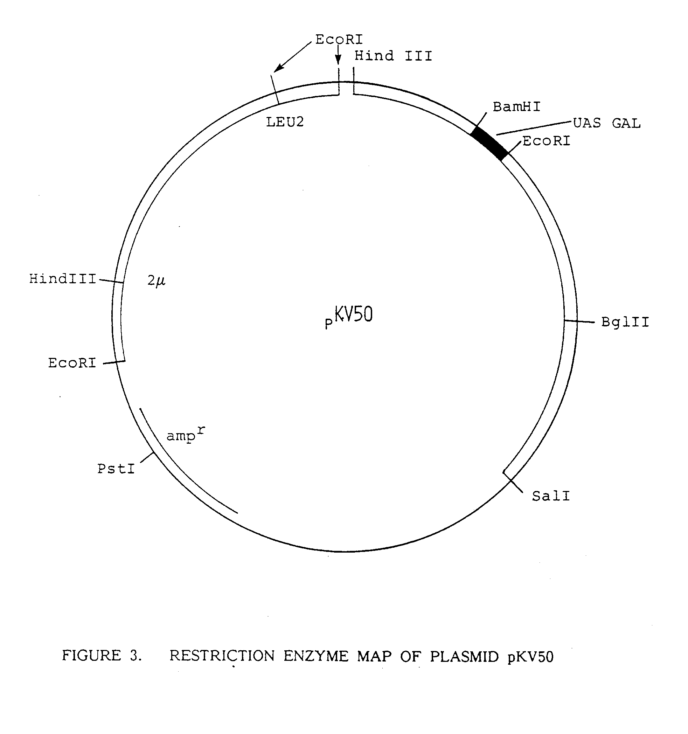

The deletion derivative above was further modified by the attachment of the 3′ transcription terminator sequence of the PGK gene. This was accomplished by digestion of the plasmid with BglII and PstI and then ligating the large fragment thus generated with the small BglII-PstI fragment containing the 3′ transcription terminator of the PGK gene derived from plasmid pMA91 (Mellor et al., 1983). The resultant plasmid was further modified by insertion of the GAL10 UAS. To this end, the BglII-BamHI fragment from pDB2 (Figure 2) was isolated and inserted at the unique BamHI site, yielding plasmid pKV50 (Figure 3). The orientation of the UAS site in this latter plasmid is such that the reformed BamHI site is distal to the PGK promoter.

### Cloning of CYC-1 Promoter and Deletion of UAS Sequences

The cytochrome C-1 gene and its 5′ and 3′ flanking regions have been cloned and their DNA sequence determined (Smith et al., 1979). An expression vector based upon these flanking regions has already been described (Ernst and Chan, 1985) and is shown in Figure 3. This plasmid, pEX-2, contains the promoter and terminator sandwiched between an EcoR1-SmaI-BamHI cloning linker, into which heterologous genes may be inserted for expression. The promoter region does not contain an initiation codon as the promoter is derived from the CYC1-13 mutant, in which this latter codon has been mutated to ATA. Accordingly, translation will commence at the first initiation codon 3′ to this position.

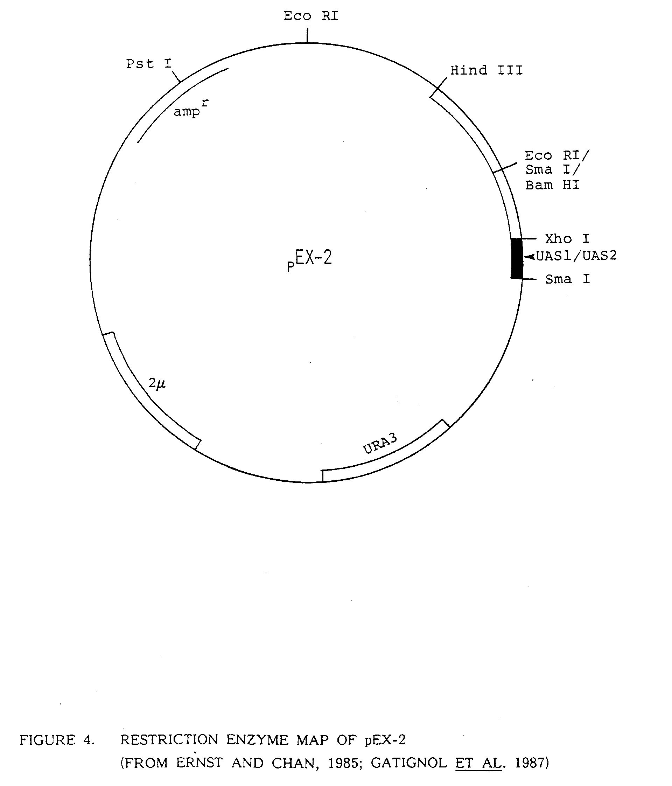

The positions of the UAS in the 5′ region are known. These lie between the XhoI and SmaI sites, indicated in Figure 4 (McNeill and Smith, 1986). As shown, the 0.5kb HindIII-XhoI fragment from pEX-2 will encompass the transcription terminator, the cloning linker and the promoter but not the UAS sequences.

### The Construction of the Bidirectional Promoter

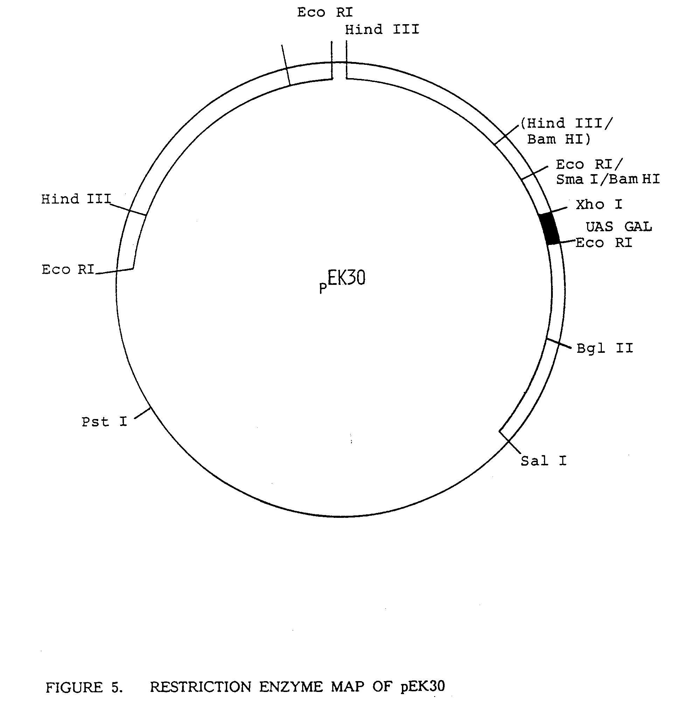

This may be accomplished by placing the 0.5kb HindIII-XhoI fragment described above adjacent to the GAL10 UAS in pKV50 distal to the PGK promoter and reading the opposite way from the PGK promoter. Thus, pKV50 is digested with BamHI and made flush ended by treatment with Klenow polymerase. Next, the 0.5kb fragment is ligated to this species, having itself been made flush ended also (Figure 4). The promoter-containing fragment may be inserted in either orientation; the correctly orientated clone, i.e. with the terminator distal to the UAS, is shown in Figure 5 and is termed pEK30.

### Induction of Two Heterologous Proteins via Bidirectional Promoter

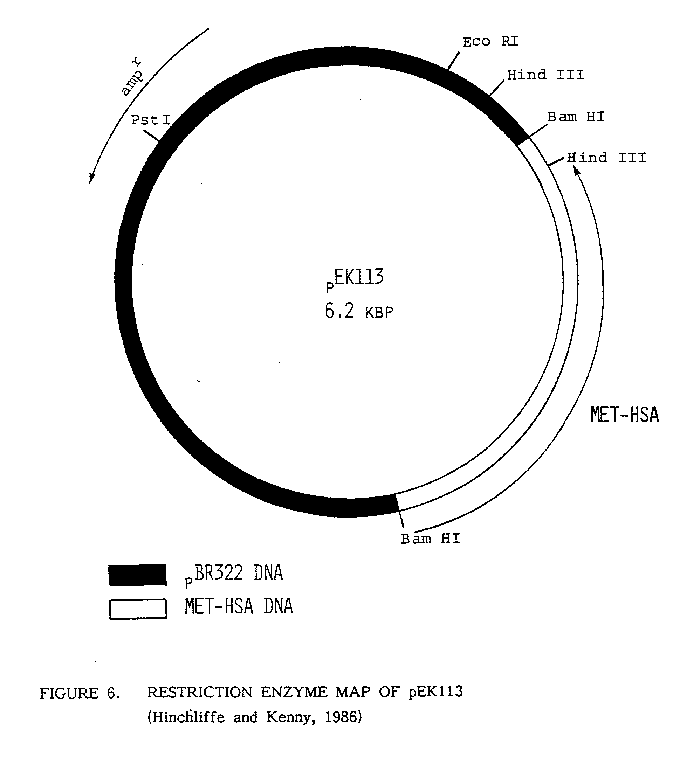

The utility of the bidirectional promoter was demonstrated by the expression of HSA and pro-urokinase. The HSA gene was constructed as described by Hinchliffe and Kenny (1986) and was obtained from plasmid pEK113, shown in Figure 6. The gene for pro-urokinase was isolated from plasmid pDBUro1 (Figure 7). This plasmid contains the pro-urokinase structural gene cDNA, cloned as described by Heyneker et al., (1986). The DNA sequence in pDBUro1, however, has been modified at the N-terminal coding region to encode methionyl-pro-urokinase, such that the mature gene product can be expressed from transcriptional fusion such as that produced by pEX-2 or pKV50 (supra) that do not themselves possess initiator codons. The sequence of this portion of the gene is shown in Figure 7.

### Subcloning of HSA

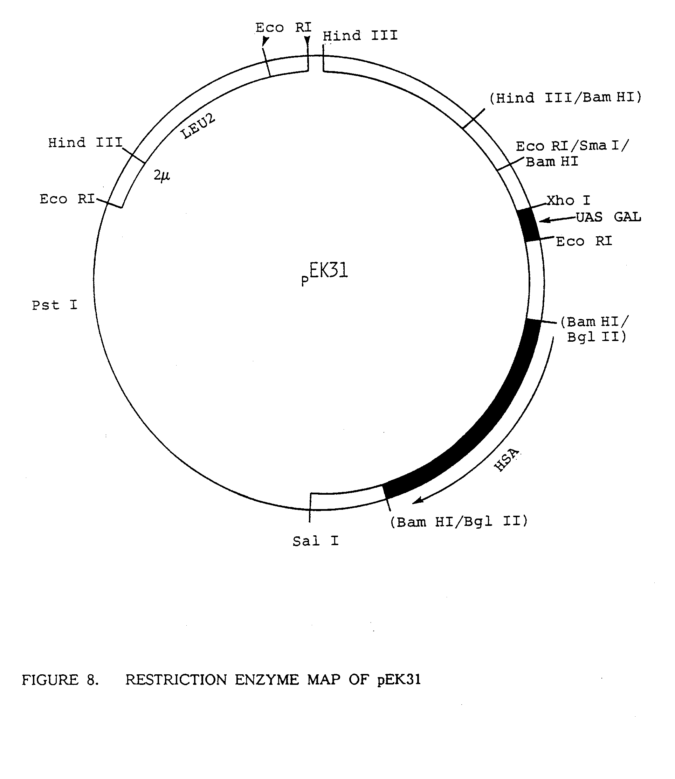

The 1.8kb BamHI fragment from pEK113 (Hinchliffe and Kenny, 1986) was isolated and cloned into the BglII site of pEK30 to give plasmid pEK31 (Figure 8).

### Subcloning of Pro-urokinase

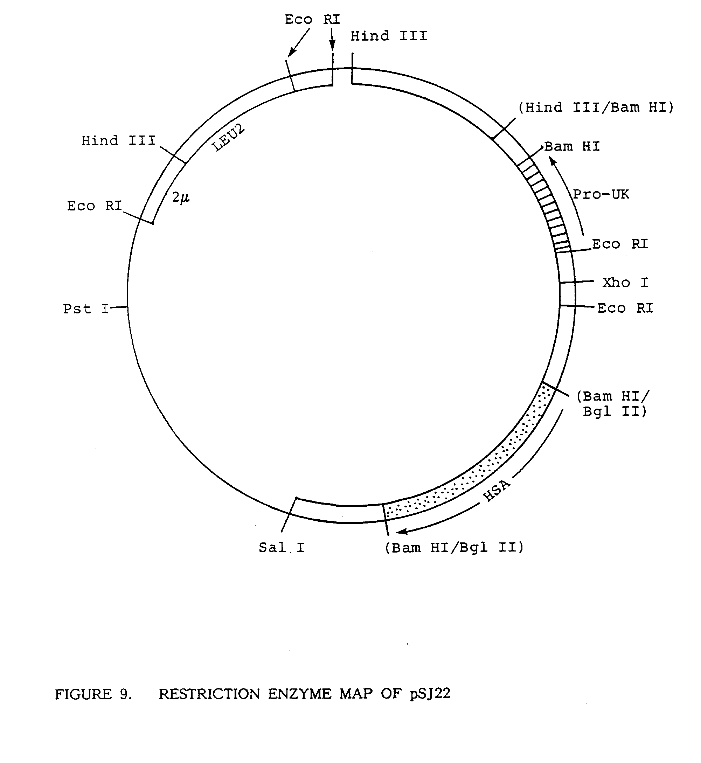

Plasmid pEK31 was digested with SmaI and ligated to the 1.6kb HindIII-SacI fragment from pDBUro1, whose ends had been made flush by treatment with T4 DNA polymerase. SacI leaves a 3′ extension which is removed in this reaction whilst the 5′ extension is filled in. The fragment and vector were ligated to yield plasmid pSJ22, as shown in Figure 9.

The plasmid pSJ22 was transformed into the yeast strain DBY745 selected for leucine prototroph. A single transformant DBY745 (pSJ22) was analysed for HSA and pro-urokinase production as follows.

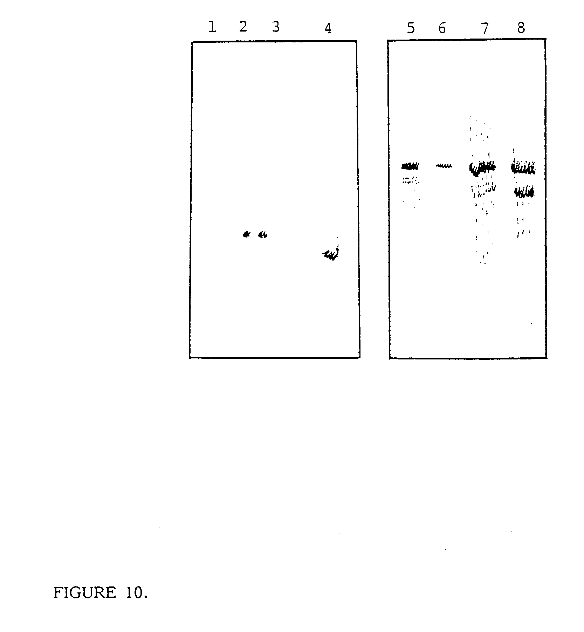

The strain was cultured in minimal medium, as described by Hawthorne and Mortimer (1960), containing 2% w/v glucose as a carbon source until the OD₆₀₀ reached 1.0. At this point the culture was harvested, a small sample removed and the remainder resuspended to the same OD₆₀₀ in minimal medium supplemented with 2% w/v galactose as a carbon source. Incubation was continued for a further 24 hours, after which time the culture was harvested and cell extracts prepared both from the induced culture and the uninduced sample. The protein components of the extracts were resolved by polyacrylamide gel electrophoresis and immunoblotted Hinchliffe and Kenny, 1986) The results are shown in Figure 10. The samples were loaded in duplicate and one set probed with urokinase antibodies and the other probed with HSA antibodies. Standards for both proteins were included. As shown, pro-urokinase is not detectable in the pre-induced sample and only small levels of HSA are observed in the same sample. Upon induction, pro-urokinase is revealed and HSA expression substantially increased above background level.

These data demonstrate the utility of the bidirectional expression system.

### EXAMPLE 2 : BREWING YEAST

Similar results may be obtained for industrial yeast strains, in particular brewing yeats. However, in the latter case one may first introduce a suitable selectable marker for transformant selection, such as the CUP-1 gene which confers resistance to elevated levels of copper ions (Henderson, et al., 1985).

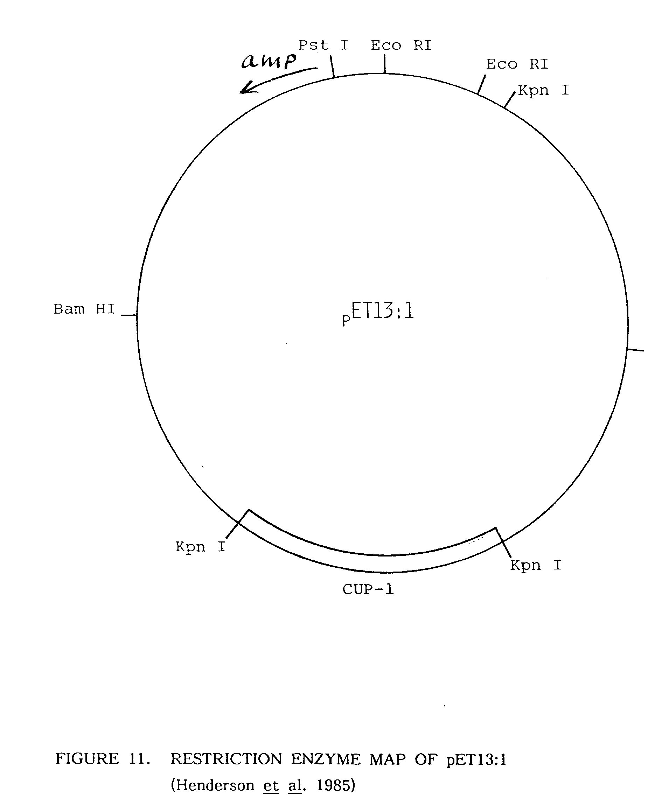

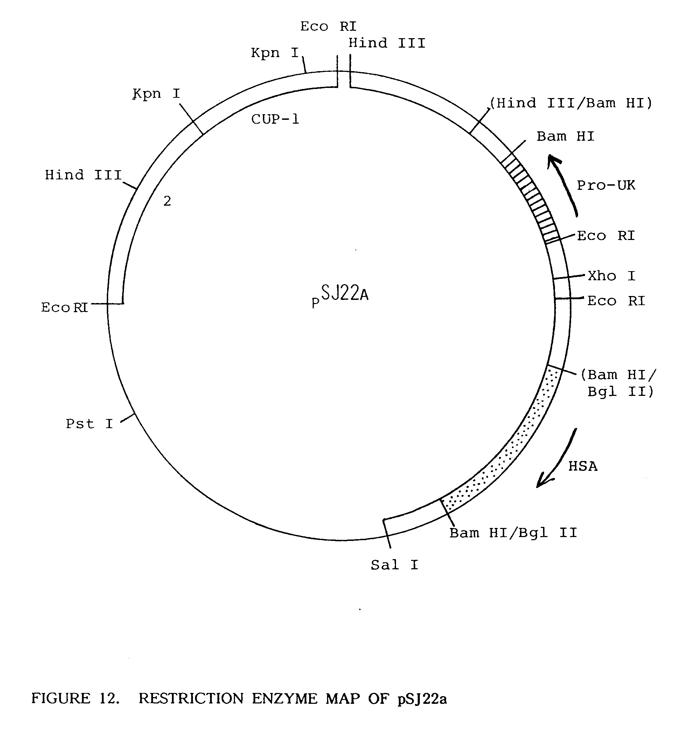

The CUP-1 gene may be obtained from plasmid pET13:1 (Henderson et al., 1985) whose structure is shown diagrammatically in Figure 11. This encodes the CUP-1 gene on a 2Kb KpnI fragment. Accordingly, plasmid pSJ22 can be digested with KpnI and ligated with the above fragment to yield pSJ22a, as shown in Figure 12. This plasmid may next be transformed into strain BB10.2, selected for copper-resistance as described by Hinchliffe and Kenny (1986).

### EXAMPLE 3

### Intracellular Expression of Pro-Urokinase

In this Example, a plasmid was constructed utilising the bidirectional expression system which allowed the induced synthesis of HSA and pro-urokinase such that the latter was retained intracellularly as in Example 1 but HSA was secreted to the culture medium. This was accomplished by emplacing the yeast MFalpha-1 gene signal sequence (pre-pro sequence) immediately 5′ to the structural gene for HSA

### Construction of MFalpha-1 Pre-Pro Coding Sequence

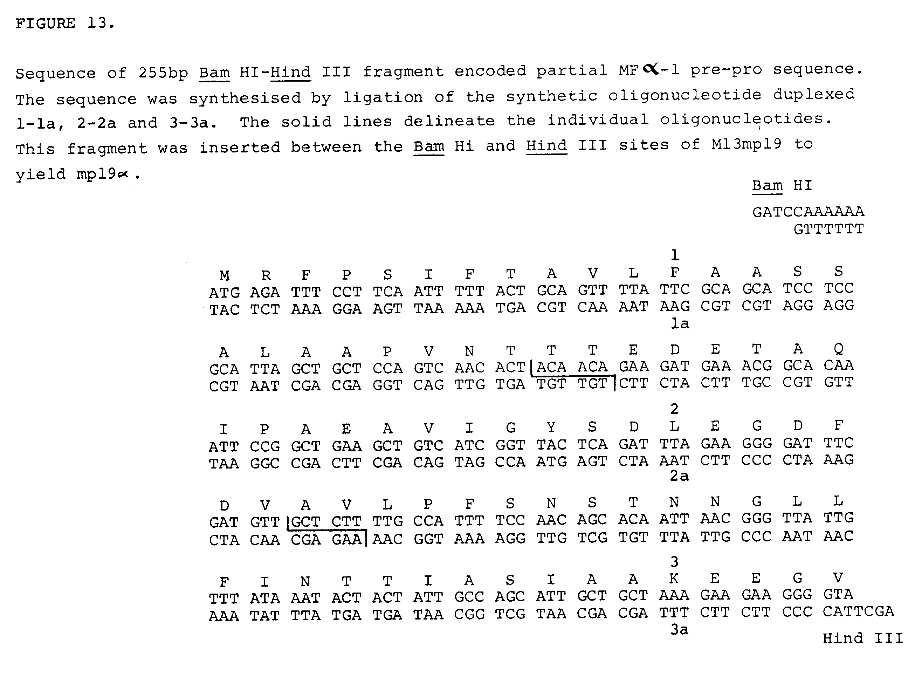

The MFalpha-1 pre-pro sequence was constructed according to the published sequence (Piggott et al., 1987, Current Genetics, 12, 561-567) as a series of oligonucleotides, as shown in Figure 13. These were assembled according to the method described by Heaphy et al.,

Protein Engineering, 1, 425-431, 1987. The oligonucleotides were first enzymatically phosphorylated, mixed and ligated as described by Heaphy et al. Oligonucleotides 1 and 3a were left non-phosphorylated. Following ligation, the desired fragment was cloned into M13mp19 (Messing, 1983, Methods in Enzymology, 101, 20-78) between the BamHI and HindIII sites to yield mp19alpha.

### Attachment of MFalpha-1 Pre-Pro Sequence to HSA

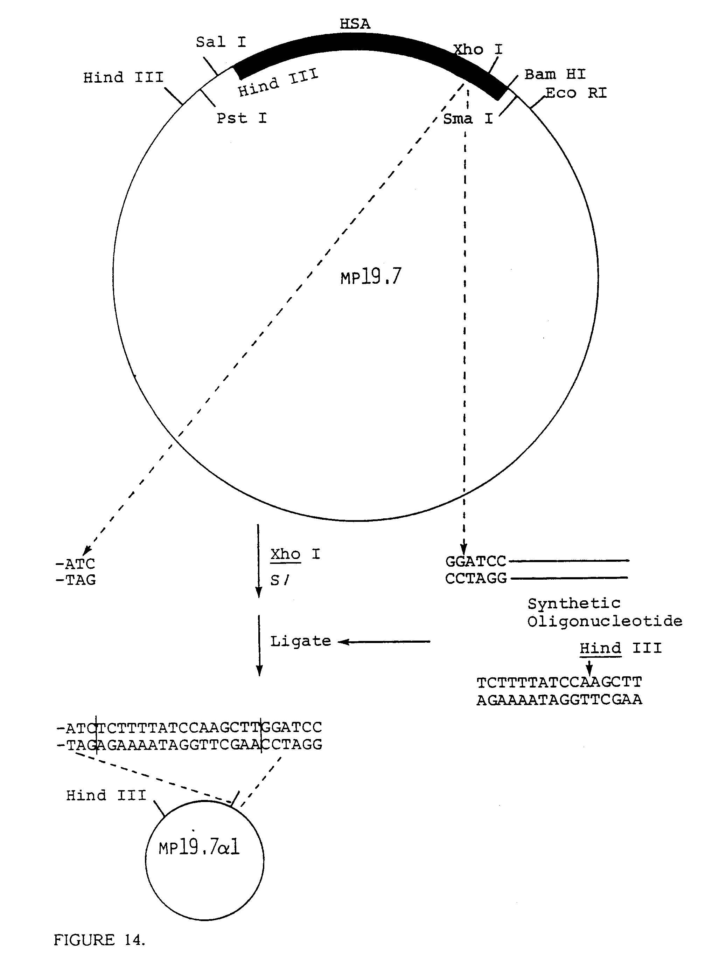

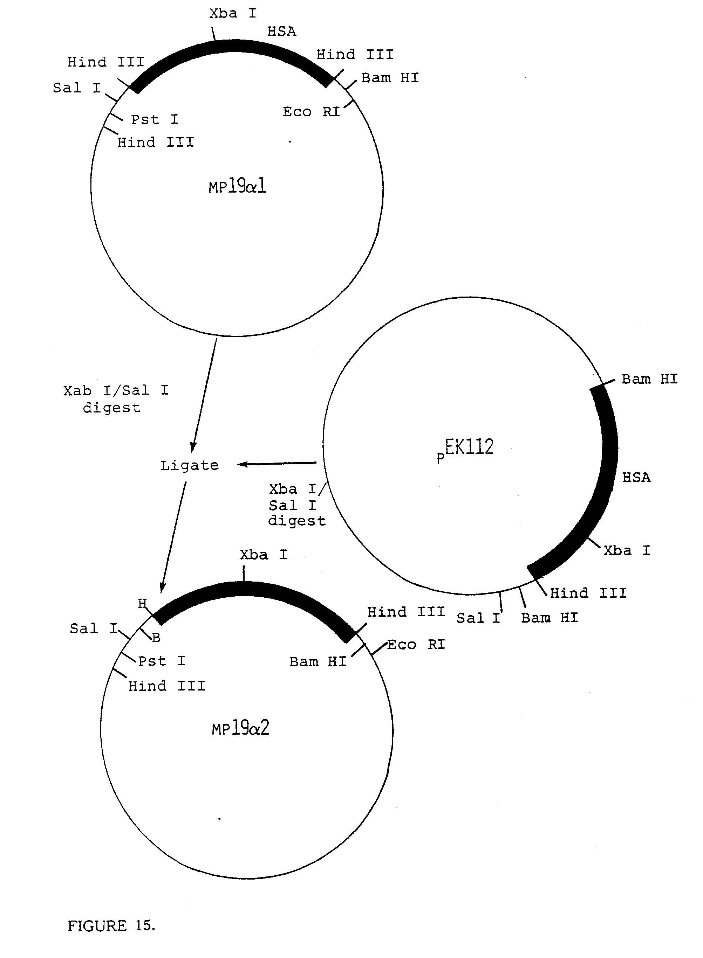

First, mp19.7 (Hinchliffe and Kenny, 1986) was digested with XhoI and S1 nuclease and ligated with a synthetic oligonucleotide to yield mp19.7alpha1 (Figure 14). This latter molecule was next digested with XbaI and SalI and ligated with pEK112 which had been similarly digested to yield mp19.7alpha2, as shown in Figure 15. pEK112 is essentially identical to pEK113 (Hinchliffe and Kenny, 1986) except the insert encoding the HSA is in the opposite orientation in the former molecule.

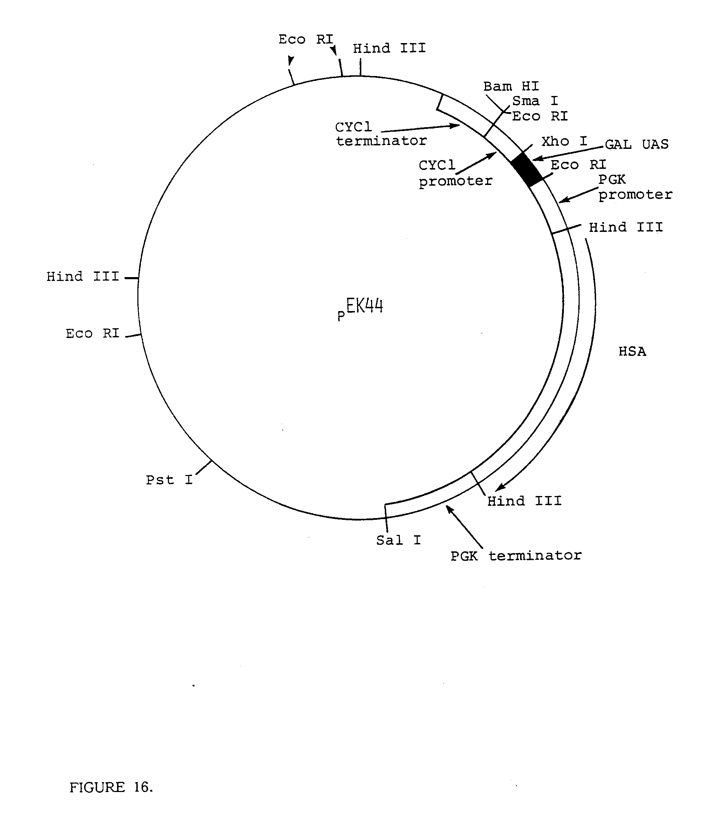

This substitution placed a BamHI site 3′ to the HSA structural gene sequence. Finally, the 0.25kb BamHI-HindIII fragment from mp19alpha was ligated to the 1.8kb BamHI-HindIII fragment from mp19alpha2 and this mixture ligated with BglII digested pEK30 to yield pEK44 (Figure 16).

### Cloning of the Pro-Urokinase Gene in pEK44

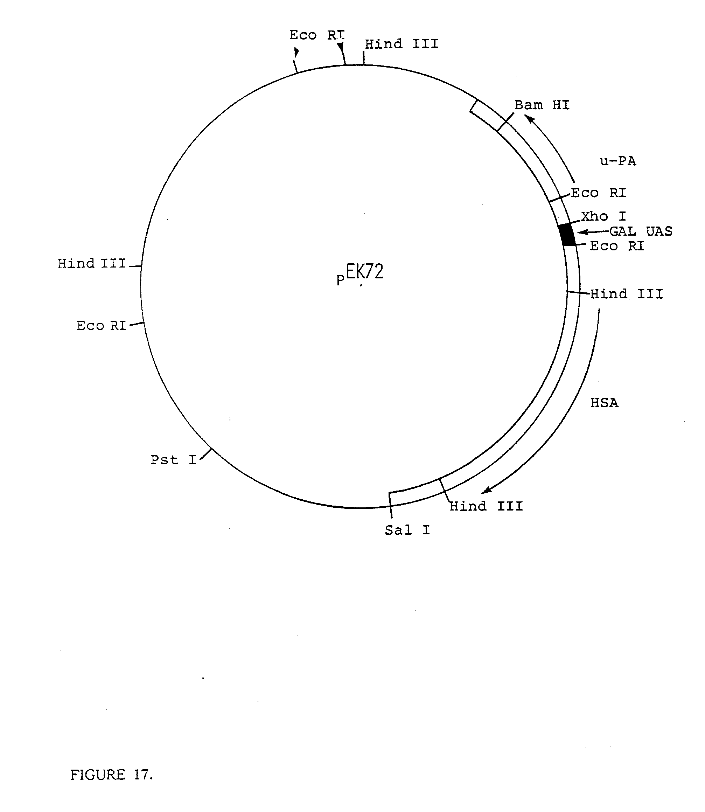

Plasmid pEK44 was digested with SmaI and ligated with the blunt ended SacI-HindIII fragment from pDBUrol which encodes Met-u-PA. This yielded the bidirectional expression plasmid pEK72 (Figure 17). This is essentially identical to plasmid pSJ22 save that the gene encoding mature HSA is preceded by the MFalpha-1 pre-pro sequence.

### Analysis of Transformants

Plasmids pEK44 and pEK72 were transformed into the host yeast DBY745 and extracts prepared of both induced and non-induced cultures as described previously. In this case, however, the intracellular fraction was analysed for pro-urokinase and the culture medium for HSA using the techniques of SDS electrophoresis and Western blotting. The results obtained were comparable to and agreed with the earlier results for pSJ22. pEK72 in the absence of galactose directed a low level of secretion of HSA, which increased markedly with galactose.

The levels of HSA secreted by pEK72 and pEK44 transformants were similar. Pro-urokinase was not detected in intracellular extracts of pEK44 cultures or non-induced pEK72 cultures and could only be detected in pEK72 cultures induced with galactose.

These data further show the utility of the bidirectional promoter system.

Further aspects of the invention include the novel plasmids described above, including pKV50, pEK30, pEK31, pSJ22 and pSJ22a, and yeast (particularly brewer's yeast) transformed with pSJ22a.

### REFERENCES

Dobson et al, (1982), Nucleic Acids Res., 10, 2625-2637.
Gatignol, A., Baron, M and Tiraby, G. (1987) Mol. Gen. Genet. 207, 342-348.
Giniger, E., Varnum, S.M. and Ptashne, M. (1985), Cell 40, 767-774.
Gorman, C. (1986) in "DNA Cloning" Volume III, (Ed.) Glover, D., IRL Press, Oxford.
Guarante, L., Yocum, R.R., and Gifford, P. (1982) Proc. Natl. Acad. Sci. USA 79, 7410-7414.
Hawthorne, D.C. and Mortimer, R.K. (1960) Genetics 45, 1085-1110.
Heaphy et al, (1987) Protein Engineering 1, 425-431.
Henderson, R.C.A., Cox, B.S. and Tubb, R. (1985), Curr. Genet. 9, 133.
Heyneker, H.L., Holmes, W.E. and Vehar, G.A. (1986) U.K. Patent 2121050B.
Hinchliffe, E. and Kenny, E. (1986) EP-A-201 239 Hopper, J.E., Broach, J.R., and Rowe, L.B. (1978) Proc. Natl. Acad. Sci. USA 75, 2878-2882.
Kakidani, H. and Ptashne, M. (1988) Cell, 52, 161-167. Kingsman, S.M., Kingsman A.J., and Mellor, J. (1986) TIBTECH 5, 53-57.
Maniatis, T., Fritsch, E.F. and Sambrook, J. (1982) Molecular Cloning: A Laboratory Manual. Cold Spring Harbor Laboratory, Cold Spring Harbor, N.Y. USA.
McNeill, J.B., and Smith, M. (1986) J. Mol. Biol. 187, 363-378.
Mellor, J., Dobson, M.J., Roberts, N.A., Tuite, M.F., Emtage, J.S., White, S., Lowe, P.A., Patel, T., Kingsman, A.J. and Kingsman, S.M. (1983) Gene, 24, 1-14.
Messing, (1983) Methods in Enzymology 101, 20-78.
Piggott et al (1987) Current Genetics 12, 561-567.
Scheidereit, C. et al, (1983) Nature 304, 749-752.
Smith, M., Leung, D.W., Gillam, S., and Astell, C.R., (1979) Cell, 16, 753-761.
St. John, T., Schrerer, S., McDonnell, M., and David, R. 1981) J. Mol.Biol, 152, 317-334.
St. John, T., and David, R. (1981) J. Mol. Biol. 152, 285-315.
St. John, T., and Davis, R. (1979) Cell 16, 443-452. Struhl, K. (1984) Proc. Natl. Acad. Sci USA 81, 7865-7869 Webster, N. et al (1988) Cell, 52, 169-178
West, R.W. et al., (1984) Mol. Cell. Biol. 4, 2467-

## Claims (Claims for the following Contracting State(s): AT, BE, CH, DE, FR, GB, GR, IT, LI, LU, NL, SE)

1. A DNA sequence comprising the GAL UAS located between first and second transcription promoters, the said two promoters each being heterologous to the said UAS, in mutually opposite orientation and operably linked to the said UAS such that the UAS is capable of activating transcription from each said promoter, each said promoter being derived from a gene which has a native UAS, the said native UAS being absent in the said DNA sequence.

2. A sequence according to Claim 1 and additionally comprising two coding regions respectively lying on the side of the two promoters remote from the GAL UAS and in correct reading frame with the promoters, provided that the coding regions are not respectively those for GAL1 and GAL10.

3. A sequence according to Claim 2 wherein the coding regions respectively encode human serum albumin (HSA) and urokinase.

4. A sequence according to Claim 3 wherein one said coding region comprises at its 5' end an initial region encoding an amino acid sequence so chosen that the polypeptide translated from the said coding region will be joined at its N-terminal to the said amino acid sequence and secreted from a host cell in which the DNA sequence is located for transcription and translation.

5. A sequence according to any one of the preceding claims wherein the GAL UAS comprises multiple copies of any one or more of the four homologous 17 base pair sequences which bind GAL4 protein in yeast.

6. A vector comprising a sequence according to any one of the preceding claims and suitable for transforming a microorganism.

7. A plasmid according to Claim 6 and capable of self-replication in yeast.

8. A yeast cell transformed with a plasmid according to Claim 7.

9. A process for producing two or more polypeptides by fermentation of a yeast cell according to Claim 8 in a suitable medium including sufficient galactose to activate the GAL UAS.

## Claims (Claims for the following Contracting State(s): ES)

1. A process for preparing a DNA sequence comprising locating, by recombinant DNA techniques, the GAL UAS between first and second transcription promoters, the said two promoters each being heterologous to the said UAS, in mutually opposite orientation and operably linked to the said UAS such that the UAS is capable of activating transcription from each said promoter, each said promoter being derived from a gene which has a native UAS, the said native UAS being absent in the said DNA sequence.

2. A process according to Claim 1 additionally providing two coding regions to lie respectively on the side of the two promoters remote from the GAL UAS and in correct reading frame with the promoters, except that the coding regions are not respectively those for GAL1 and GAL10.

3. A process according to Claim 2 wherein the coding regions respectively encode human serum albumin (HSA) and urokinase.

4. A process according to Claim 3 wherein one said coding region comprises at its 5' end an initial region encoding an amino acid sequence so chosen that the polypeptide translated from the said coding region will be joined at its N-terminal to the said amino acid sequence and secreted from a host cell in which the DNA sequence is located for transcription and translation.

5. A process according to any one of the preceding claims wherein the GAL UAS comprises multiple copies of any one or more of the four homologous 17 base pair sequences which bind GAL4 protein in yeast.

6. A process for preparing a vector comprising joining, by recombinant DNA techniques, a sequence prepared according to any one of the preceding claims and vector DNA suitable for transforming a microorganism.

7. A process according to Claim 6 wherein the vector is capable of self-replication in yeast.

8. A process for transforming a yeast cell comprising introducing into the yeast cell a vector prepared according to Claim 7.

9. A process for producing two or more polypeptides by fermentation of a yeast cell prepared according to Claim 8 in a suitable medium including sufficient galactose to activate the GAL UAS.

## Patentansprüche (Patentansprüche für folgende(n) Vertragsstaat(en): AT, BE, CH, DE, FR, GB, GR, IT, LU, NL, SE)

1. DNA-Sequenz, enthaltend die zwischen ersten und zweiten Transkriptionspromotor angeordnete GAL UAS, wobei die beiden Promotoren jeweils heterolog zu der UAS sind, sich zueinander in entgegengesetzter Orientierung befinden und unter Erhaltung der Funktionsfähigkeit mit der UAS derart verknüpft sind, daß die UAS die Transkription von jedem dieser Promotoren aus aktivieren kann, wobei jeder Promotor aus einem Gen mit einer nativen UAS stammt und diese native UAS in der DNA-Sequenz nicht vorhanden ist.

2. Sequenz nach Anspruch 1, die zusätzlich zwei codierende Regionen enthält, welche jeweils auf der von der GAL UAS abgewandten Seite der beiden Promotoren liegen und sich im korrekten Leserahmen mit den Promotoren befinden, unter der Bedingung, daß die codierenden Regionen nicht diejenigen für GAL1 bzw. GAL10 sind.

3. Sequenz nach Anspruch 2, worin die codierenden Regionen für Humanserumalbumin (HSA) bzw. Urokinase codieren.

4. Sequenz nach Anspruch 3, worin eine der codierenden Regionen an ihrem 5'-Ende eine für eine Aminosäuresequenz codierende Anfangsregion aufweist, die so gewählt ist, daß das aus dieser codierenden Region translatierte Polypeptid an seinem N-Terminus mit dieser Aminosäuresequenz verknüpft ist und aus einer Wirtszelle, in welcher sich die DNA-Sequenz für die Transkription und die Translation befindet, sezerniert wird.

5. Sequenz nach einem der voranstehenden Ansprüche, worin die GAL UAS mehrfache Kopien von einer oder mehreren der vier homologen, 17 Basenpaare langen Sequenz(en) umfaßt, die GAL4-Protein in Hefe binden.

6. Vektor, enthaltend eine Sequenz nach einem der voranstehenden Ansprüche, der sich für die Transformierung eines Mikroorganismus eignet.

7. Plasmid nach Anspruch 6, welches in der Lage ist, sich in Hefe selbst zu replizieren.

8. Hefezelle, die mit einem Plasmid nach Anspruch 7 transformiert ist.

9. Verfahren zum Herstellen von zwei oder mehreren Polypeptiden durch Fermentierung einer Hefezelle nach Anspruch 8 in einem geeigneten Medium, das ausreichend Galaktose enthält, um die GAL UAS zu aktivieren.

## Patentansprüche (Patentansprüche für folgende(n) Vertragsstaat(en): ES)

1. Verfahren zum Herstellen einer DNA-Sequenz, umfassend das Anordnen der GAL UAS zwischen ersten und zweiten Transkriptionspromotoren, und zwar mittels der Techniken mit rekombinanter DNA, wobei die beiden Promotoren jeweils heterolog zu der UAS sind, sich zueinander in entgegengesetzter Orientierung befinden und unter Erhaltung der Funktionsfähigkeit mit der UAS derart verknüpft sind, daß die UAS die Transkription von jedem dieser Promotoren aus aktivieren kann, wobei jeder Promotor aus einem Gen mit einer nativen UAS stammt und diese native UAS in der DNA-Sequenz nicht vorhanden ist.

2. Verfahren nach Anspruch 1, durch welches weiterhin zwei codierende Regionen auf der jeweils von der GAL UAS abgewandten Seite der beiden Promotoren und im korrekten Leserahmen mit den Promotoren angeordnet werden, mit der Ausnahme, daß die codierenden Regionen nicht diejenigen für GAL1 bzw. GAL10 sind.

3. Verfahren nach Anspruch 2, worin die codierenden Regionen für Humanserumalbumin (HSA) bzw. Urokinase codieren.

4. Verfahren nach Anspruch 3, worin eine der codierenden Regionen an ihrem 5'-Ende eine für eine Aminosäuresequenz codierende Anfangsregion aufweist, die so gewählt ist, daß das aus dieser codierenden Region translatierte Polypeptid an seinem N-Terminus mit dieser Aminosäuresequenz verknüpft ist und aus einer Wirtszelle, in welcher sich die DNA-Sequenz für die Transkription und die Translation befindet, sezerniert wird.

5. Verfahren nach einem der voranstehenden Ansprüche, worin die GAL UAS mehrfache Kopien von einer oder mehreren der vier homologen, 17 Basenpaare langen Sequenz(en) umfaßt, die GAL4-Protein in Hefe binden.

6. Verfahren zum Herstellen eines Vektors, umfassend das Verknüpfen einer nach einem der voranstehenden Ansprüche hergestellten Sequenz mit einer Vektor-DNA, die sich für die Transformierung eines Mikroorganismus eignet, und zwar mit Hilfe der Techniken mit rekombinanter DNA.

7. Verfahren nach Anspruch 6, worin der Vektor sich in Hefe selbst replizieren kann.

8. Verfahren zum Transformieren einer Hefezelle, umfassend das Einführen eines nach Anspruch 7 hergestellten Vektors in die Hefezelle.

9. Verfahren zum Herstellen von zwei oder mehreren Polypeptiden durch Fermentierung einer nach Anspruch 8 hergestellten Hefezelle in einem geeigneten Medium mit ausreichend Galaktose, um die GAL UAS zu aktivieren.

## Revendications (Revendications pour l'(les) Etat(s) contractant(s) suivant(s): AT, BE, CH, DE, FR, GB, GR, IT, LI, LU, NL, SE)

1. Séquence d'ADN comprenant l'UAS GAL localisée entre le premier et le deuxième promoteur de transcription, lesdits deux promoteurs étant chacun hétérologue à ladite UAS, en orientation mutuellement opposée et liés de manière fonctionnelle à ladite UAS de telle sorte que l'UAS soit susceptible d'activer la transcription à partir de chacun desdits promoteurs, chacun desdits promoteurs étant dérivé d'un gène ayant une UAS native, ladite UAS native étant absente de ladite séquence d'ADN.

2. Séquence selon la revendication 1 et comprenant en outre deux régions codantes situées respectivement du côté des deux promoteurs éloigné de l'UAS GAL et dans la bonne phase de lecture par rapport aux promoteurs, à condition que les régions codantes ne soient pas respectivement celles pour GAL1 et GAL10.

3. Séquence selon la revendication 2, caractérisée en ce que les régions codantes codent respectivement pour la sérum albumine humaine (HSA) et l'urokinase.

4. Séquence selon la revendication 3, caractérisée en ce que l'une desdites régions codantes comprend à son extrémité 5' une région initiale codante pour une séquence d'acides aminés choisie de telle sorte que le polypeptide traduit à partir de ladite région codante sera joint à son extrémité N-terminale à ladite séquence d'acides aminés et sécrété à partir d'une cellule hôte dans laquelle la séquence d'ADN est localisée pour la transcription et la traduction.

5. Séquence selon l'une quelconque des revendications précédentes, caractérisée en ce que l'UAS GAL comprend des copies multiples de l'une quelconque ou de plusieurs des quatre séquences homologues de 17 paires de base qui lient la protéine GAL4 chez la levure.

6. Vecteur comprenant une séquence selon l'une quelconque des revendications précédentes et approprié pour la transformation d'un microorganisme.

7. Plasmide selon la revendication 6 et susceptible d'autoréplication chez la levure.

8. Cellule de levure transformée par un plasmide selon la revendication 7.

9. Procédé de production de deux ou plusieurs polypeptides par fermentation d'une cellule de levure selon la revendication 8 dans un milieu approprié renfermant suffisamment de galactose pour activer l'UAS GAL.

## Revendications (Revendications pour l'(les) Etat(s) contractant(s) suivant(s): ES)

1. Procédé de préparation d'une séquence d'ADN comprenant la localisation, par les techniques d'ADN recombinant, de l'UAS GAL entre le premier et le deuxième promoteur de transcription, lesdits deux promoteurs étant chacun hétérologue à ladite UAS, en orientation mutuellement opposée et liés de manière fonctionnelle à ladite UAS de telle sorte que l'UAS soit susceptible d'activer la transcription à partir de chacun desdits promoteurs, chacun desdits promoteurs étant dérivé d'un gène ayant une UAS native, ladite UAS native étant absente de ladite séquence d'ADN.

2. Procédé selon la revendication 1, fournissant en outre deux régions codantes devant se situer respectivement du côté des deux promoteurs éloigné de l'UAS GAL et dans la bonne phase de lecture par rapport aux promoteurs, sauf que les régions codantes ne sont pas respectivement celles pour GAL1 et GAL10.

3. Procédé selon la revendication 2, caractérisé en ce que les régions codantes codent respectivement pour la sérum albumine humaine (HSA) et l'urokinase.

4. Procédé selon la revendication 3, caractérisé en ce que l'une desdites régions codantes comprend à son extrémité 5' une région initiale codante pour une séquence d'acides aminés choisie de telle sorte que le polypeptide traduit à partir de ladite région codante sera joint à son extrémité N-terminale à ladite séquence d'acides aminés et sécrété à partir d'une cellule hôte dans laquelle la séquence d'ADN est localisée pour la transcription et la traduction.

5. Procédé selon l'une quelconque des revendications précédentes, caractérisé en ce que l'UAS GAL comprend des copies multiples de l'une quelconque ou de plusieurs des quatre séquences homologues de 17 paires de base qui lient la protéine GAL4 chez la levure.

6. Procédé de préparation d'un vecteur comprenant la jonction, par les techniques d'ADN recombinant, d'une séquence préparée selon l'une quelconque des revendications précédentes et de l'ADN vecteur approprié pour la transformation d'un microorganisme.

7. Procédé selon la revendication 6, caractérisé en ce que le vecteur est susceptible d'autoréplication chez la levure.

8. Procédé de transformation d'une cellule de levure comprenant l'introduction dans la cellule de levure d'un vecteur préparé selon la revendication 7.

9. Procédé de production de deux ou plusieurs polypeptides par fermentation d'une cellule de levure préparée selon la revendication 8 dans un milieu approprié renfermant suffisamment de galactose pour activer l'UAS GAL.
